# EUROPEAN PATENT APPLICATION

(11) **EP 3 427 833 A1**
(43) Date of publication of application: **16.01.2019**
(21) Application number: 18000584.5
(22) Date of filing: 09.07.2018
(51) Int. Cl.: B01L 3/14, G01N 33/49, A61B 5/154

(54) **A DEVICE FOR TEST PREPARATION OF BLOOD FOR DETERMINATION OF GLUCOSE CONCENTRATION IN BLOOD PLASMA**

(30) Priority: 10.07.2017 RU 2017124460
(71) Applicant: Lind, Victor, 21004 Narva (EE); Fedotov, Maxim, 21004 Narva (EE)
(72) Inventor: Lind, Victor, 21004 Narva (EE); Fedotov, Maxim, 21004 Narva (EE); Dorofeikov, Vladimir, 21004 Narva (EE)
(74) Representative: Koitel, Raivo

(57) **Abstract**

The device for blood sample preparation for determining the concentration of glucose in the blood plasma relates to medicine, more specifically to clinical laboratory diagnostics and endocrinology, and can be used for the diagnosing of diabetes mellitus.

The device is a vacuum vial with a bar code and a protective cover of grey colour assembled with a rubber stopper comprising an inert separation gel in an amount of 0.6-1.2 g at the bottom and a mixture of potassium oxalate with sodium fluoride on the walls of the vial.

The device provides increased accuracy of determining the glucose concentration in blood plasma, reduces the time of sample preparation, reduces the risk of haemolysis during transportation and storage of blood, and reduces the risk of infection of laboratory personnel with blood-borne infections.

## Description

### Technical field

The invention relates to medicine, more specifically to clinical laboratory diagnostics and endocrinology, and can be used for diagnosing of diabetes mellitus.

### Prior Art

During the past 10-20 years, the prevalence of diabetes mellitus, impaired glucose tolerance and especially gestational diabetes mellitus (GDM) worldwide as well as in our country has been steadily increasing. GDM is a disease characterized by hyperglycaemia that is first detected during pregnancy, but that does not meet the criteria of "manifested" (newly diagnosed) gestational diabetes. An international multi-centre "Hyperglycaemia and Adverse Pregnancy Outcome" study conducted in years of 2000 demonstrated that the GDM diagnosis criteria need to be revised. So, the adverse pregnancy outcomes among the observed women showed direct proportional increase starting with a significantly lower level of glycaemia than previously accepted as a criterion for existence of GDM. Based on the results of a study involving over 23 000 pregnant women undergoing a 75-gram oral glucose tolerance test (OGTT), the Implementation of the International Association of Diabetes and Pregnancy Study Groups IADPSG in USA proposed new criteria for diagnosing GDM in 2008. The study showed that women whose OGTT glucose result in venous blood plasma exceeded at least one threshold point experienced preeclampsia and had large children twice as much. These pregnant women also had a 45% increase in the frequency of preterm delivery and need for C-section. During 2010-2012, a number of developed countries, including the United States, Japan, Germany, Israel and Finland, independently adopted the new GDM diagnosing criteria based on the results of this study.

After several discussions in the given issue, the experts of the Russian Association of Endocrinologists and the Russian Society of Obstetricians and Gynaecologists also reached the conclusion that it is necessary to adopt new criteria for diagnosing GDM. Later, the Russian Consensus on diagnosis and treatment of GDM was drawn. In 2013, the clinical guidelines (treatment protocol) "Gestational diabetes mellitus: diagnosis, treatment, postnatal observation" approved by the Ministry of Healthcare of the Russian Federation were issued. These documents set the requirements for proper diagnosis and detection of hyperglycaemia (high blood glucose concentration) more clearly. There are no such clear recommendations for the non-pregnant population and thus different medical institutions determine the glucose concentration in blood plasma, in serum, in capillary blood and whole blood.

The standards for use in accredited clinical diagnostic laboratories of the EU and Russia suggests determining of glucose necessarily in blood plasma with the use of fluoride to prevent decrease in the glucose level during storage of samples. Such recommendations become especially topical in connection with centralization of the laboratory researches, i.e. testing in large clinical diagnostic laboratories, where blood samples are delivered by transport from collection points, for example, from public or private clinics. For example, the blood tests from the blood sampling point in Murmansk are delivered by air transport company "INVITRO" to a laboratory in Moscow where the glucose level is determined. Hence, the relevance of correct and timely diagnosis of diabetes and impaired glucose tolerance is very high. This especially relates to diagnosis of anomalies in carbohydrate metabolism of pregnant women.

According to the Consensus and Recommendations, the diagnosis of anomalies in carbohydrate metabolism in case of pregnancy should be carried out in two stages. Upon the first visit of the pregnant woman to an obstetrician-gynaecologist or family physician within the period of up to 24 weeks, all women should take the following tests: determination of glucose in venous blood plasma on an empty stomach or determination of glucose in venous blood plasma at any time of the day regardless of the meal or determination of the level of glycated haemoglobin (HbA1c) using the reference values certified according to the National Glycohemoglobin Standardization Program (NGSP) and standardized in accordance with the reference values adopted in DCCT (Diabetes Control and Complications Trial). If the test result corresponds to the category of manifested diabetes mellitus (DM), its type should be specified and the patient should be immediately referred to an endocrinologist for further examination. If a randomly defined plasma glucose level is less than 11.1 mmol/l, fasting venous plasma glucose level should be determined: according to the Consensus, GDM is diagnosed at fasting glucose level higher than 5.1 mmol/l but under 7.0 mmol/l; if the fasting plasma glucose level in blood plasma is below 5.1 mmol/l, all pregnant women with a higher GDM risk should be immediately referred to OGTT. In this case, it is appropriate and correct to prescribe the fasting glucose test in a specialized laboratory with blood sampling from the vein into a vial with sodium fluoride (vial with a grey cover intended for determination of glucose concentration). Analyzis of capillary blood in laboratories in such case is recommended to be carried out only in cases of urgency due to a significantly lower accuracy of the result obtained. It is important to note that according to the opinion of experienced clinicians-endocrinologist, the harm from late correct GDM diagnosis is considerably higher than the harm from overdiagnosis, since the further algorithm for treating the disease includes a diet and careful monitoring of glucose levels and other laboratory parameters in the pregnant woman. The second stage of GDM diagnosis of patients with no hyperglycaemia detected during the first stage should be OGTT tested during their 24-28 pregnancy weeks, i.e. with the 75 g glucose tolerance test that is considered to be a safe intake for detecting anomalies in carbohydrate metabolism during pregnancy. The test is executed on an empty stomach in the morning after 8-14 hours of overnight fasting. Determination of venous plasma glucose should be performed only at an in-patient laboratory with biochemical analyzers or stationary glucose analyzers. Under the 2012 Consensus, use of portable self-monitoring means (glucometers) for conducting the test is prohibited.

It should be noted that in a recent study comparing the accuracy of 43 glucometers of different manufacturers certified in the European Union (EC), only 27 met the accuracy criteria (ISO 15197), and deflection of the results from the reference stationary analyzer readings reached 14% [J Diabetes Sci Technol. 2012 Sep 1; 6 (5); 75]. As the variability of obtained results is high enough and depends on the time elapsed from blood collection until it is analyzed, accurate determination of glucose concentration is of utmost importance in diagnosis of GDM and the subsequent treatment of patients. Specialists are well aware that blood erythrocytes use plasma glucose for supporting their vital functions and thus the plasma glucose level constantly decreases after blood sampling with every hour. This may result in over diagnosing of diabetes mellitus. When analyzing the sample at the laboratory, sodium fluoride must be added into the sample vial one hour after collection of the blood for hindering glycolysis in erythrocytes; it is recommended to transfer the blood plasma into a separate vial after blood centrifugation.

This invention improves the equipment for blood sample preparation (vacuum vials) with the purpose to accurately determine the glucose concentration in the blood of the patients.

We did a research on the patent and scientific and technical literature on the production and use of vials for blood sample preparation to determine the concentration of glucose in blood plasma.

Patents CN105193427, FUZHOU CHANGGENG MEDICAL DEVICES CO LTD, 18.08.2015; CN104510481, WENZHOU GAODE MEDICAL INSTR CO LTD, 26.09.2013; CN203169191, FU SHIMING, 18.10.2012; CN202489959, DAYONG XU, 28.02.2012; CN202430219, FU SHIMING, 23.11.2011. The patents describe blood collecting vials that comprise sodium fluoride and anticoagulants and the means of applying them to the vial walls. EDTA (ethylenediaminetetraacetate), oxalate, heparin and others are proposed as anticoagulants. The vials are used for routine blood analysis.

Patent CN202430219 describes vials with gel for extraction, purification and activation of mononuclear cells and blood lymphocytes. EDTA or citrate, heparin, Hank's Buffered Salt Solution are used as anticoagulants. Extraction of mononuclear cells increases the accuracy of the examination of patients.

Patents EP0193279, SEIKISUI CHEMICAL CO LTD, 29.01.1985; EP2548507, MATUMURA TAKAHITO, 28.12. 2010 describe vacuum vials, and manufacturing of these vials, for blood collection with inner walls coated with a hydrophilic substance preventing adhesion of the blood cells to the walls of the vial, and with a separation gel for separating the serum from the blood elements for routine blood analysis.

WO2015132774, Pasteur Institute, 07.03.2014 - Vacuum vial with anticoagulant for collecting blood and its manufacturing method.

WO02097432, HORSTI JUHA, 01.06.2001 - A vial with an anticoagulant and the method of running a blood analysis from the blood sample. EDTA is used as the anticoagulant.

US patents 5320812, 5860937, 5326535, 5246666 Becton Dickinson Company (Franklin Lakes, NJ) - patents of 1975-1999 of the oldest American company "Becton Dickinson" (BD), established in 1887, manufacturing and selling vials, instruments and reagents, including vials for blood collection. The company does business in 50 countries, employs 30 thousand persons all over the world and has subsidiaries in Ireland and Great Britain. Trademark "Vacutainer" for disposable plastic vials with dosed vacuum, intended for collection of venous blood. Vials with grey cover - for glucose analysis contain sodium fluoride and oxalate and do not contain separation gel.

These patents describe devices for collection of blood with anticoagulant with several of them having silicone coating on the walls.

US3920549, Corning Glass Works 18.11.1975 (Corning, NY) - Device for blood collection, comprises gel-like inert material with subsequent blood plasma analysis, without fluoride.

The research results by companies producing vials for glucose analysis:
- Greiner Bio-one Preanalytics (Austria) - vials for determining glucose in venous blood.
- Corway, Estonia - vials for blood collection.
- RUSTECH, Germany - systems for vacuum blood collection.

In Russia vials for glucose analysis with fluoride (grey cover) are produced by the following companies:
- OOO "Eiliton", patent No 114277 - micro-vial with a closing cover and anticoagulant for taking capillary blood samples.
- Vaku Meyer OOO "Minimed", Bryansk Oblast, found in 1992, trademark No 548792.
- Crossmed.ru - produces vials with EDTA, oxalate, heparin and sodium fluoride for studying glucose in diabetology.
- UNIVAK, trademark No 564110.

Known use of a gel-like product together with EDTA (US3920549) by applying a gel to the bottom of the vial before adding EDTA that is sprayed onto the surface of the gel. Such vial (with a lilac cover) is designed for plasma analysis, in particular for molecular diagnostics and determination of virus concentration and is not suitable for determining glucose.

Based on the analysis of patent and company documentation, we observed that for determining the glucose concentration in blood plasma, vials comprising a mixture of sodium fluoride and anticoagulant, which in most cases is EDTA (ethylenediaminetetraacetate), applied on vial walls are used - a vial with a grey cover.

As the closest analogue of the present invention, we took a vial of the company UNIVAC (ZAO "A/O Unimed", Moscow) for glucose tests with sodium fluoride and EDTA (grey cover). The vial is foreseen for studying the blood plasma in diabetology (determining the glucose level). Venous blood sample is taken with "closed" vacuum method. The vial is sterilized using the radiation method. Vial material - polyethylene terephtalate (PET). The vial cover is of grey plastic (according to the international standard ISO 6710). Stopper cover from bromobutyl rubber with a recess for multiple puncture. Filler - glycosyl sodium fluoride inhibitor and anticoagulant (EDTA) to prevent blood coagulation.

Blood sample preparation according to the closest analogue is described in the Consensus on diagnosis of GDM and includes the following procedures: after taking the patient's venous blood into a primary vacuum vial comprising anticoagulant and fluoride, the vial is thoroughly shaken to dissolve the said substances. The primary vial is then transported to the laboratory, for example by automobile or air transport.

In the laboratory, the vial is centrifuged under standard conditions, the cover is opened, the laboratory personnel removes the top layer (blood plasma) with a dispenser and transfers it to a secondary (empty) vial with the purpose to place the plasma on board of the biochemical analyzer (the plasma glucose level is stable for a long time). Beforehand, the secondary vial is numbered or a bar code is attached to it to identify the patient.

Upon preparation of blood sample for glucose analysis after centrifugation of the blood due to lack of distinct boundaries between the layers of plasma and formed elements of blood, there is a possibility of mixing layers during transportation, shaking, etc., transfer of plasma to another vial is carried out, followed by the determination of glucose concentration in it in case of the closest analogue as well as other sources of information we found. Our studies have shown that the results of such analysis are not sufficiently accurate and are also inconvenient in practical work.

The high likelihood of the destruction of erythrocytes (haemolysis) during transportation of blood vials due to shaking and the duration of whole blood storage, especially in case of elderly and weakened patients, as well as in patients with artificial heart valves, can also be attributed to the shortcomings of the closest analogue. In these test vials, the colour of the plasma turns red due to haemolysis and this is observed in 4-10% of cases. Occurrence of haemolysis in the patient's blood sample leads to the refusal of the laboratory to perform tests in such a sample (rejection of the test vials), since it causes incorrect results in treatment with biochemical analyzers. The laboratory usually considers the presence of haemolysis to be a mistake made by the medical personnel during blood collection and transportation of it and requires retesting of the patient. The drawbacks of the closest analogue include the possible mistakes in blood transferring to obtain plasma and the laboratory staff manually writing the patient's last name or number on the secondary vial placed in a biochemical analyzer (human factor) that does not provide a guarantee determination of blood glucose of the patient whose blood is delivered to the laboratory. When executing sample preparation according to the closest analogue, the possibility of contamination of personnel with blood-borne infections (manual opening of the primary vial cover, transferring of blood plasma into the secondary vial with a manual dispenser) is also possible. Additionally, it increases the working time of the laboratory personnel due to transferring of plasma from the primary vial into the secondary, which increases the cost of the analysis. At the same time, it is necessary to consider the costs of the laboratory for disposable tips of dispensers for transfer of blood plasma and the cost of secondary vials that must be disinfected and disposed of after the glucose analysis in the biochemical analyzer.

### Summary of the Invention

The technical result of the present invention lies in increased accuracy of determining the glucose concentration by drawing blood into a test vial containing separation gel at the bottom of it.

This result is achieved by the fact that in a known vacuum test vial with a barcode, a protective cover of grey colour assembled with a rubber stopper, a mixture of sodium fluoride and anticoagulant applied to the vial inner wall, followed by sterilization of the vial with radiation method, according to the given invention, additionally comprises an inert separation gel in the amount of 0.6-1.2 g, located at the bottom of the vial, as an anticoagulant-potassium oxalate.

The use of an inert separation gel (as a result of it rising up from the bottom of the vial during blood centrifugation) allows us to separate the upper layer (blood plasma) from the formed elements, with the exception of haemolysis and decrease in glucose concentration due to the inability of catabolism ("eating") of plasma glucose by erythrocytes, guaranteeing high accuracy of determining its concentration.

The use of the separation gel does not require transfer of blood plasma into another vial after centrifugation, which allows to determine the glucose concentration in the primary vial and thus reflects the true levels of glucose in the patient's blood, increasing the accuracy of determination of its concentration and reducing the time of sample preparation and also reducing the risk of infection of personnel with blood-borne infections.

The quantity of added separation gel - 0.6-1.2 g - was determined by us experimentally and multiple determination of glucose concentration demonstrated that this amount is necessary and sufficient for reliable separating the formed elements from the blood plasma under the standard procedure of centrifugation of the vial for 10 minutes with the volume of the blood sample being 3-4 ml.

Additionally, the use of the separation gel prevents the lower layer of blood comprising clots and erythrocytes getting mixed into the plasma and thus enables to determine the concentration of glucose on the automatic analyzer immediately after centrifuging the vial and prevents clogging of the needle of the biochemical analyzer sampler and its failure.

### Exemplary embodiment of the Invention

The necessity of using test vials with a gel for sample preparation of blood for glucose analysis became clear to us due to the results of the scientific research study "Study on the effect of separation gel in vacuum systems for collection and storage of blood for correct determination of glucose in blood plasma" by the federal state budgetary institution "Almazov Nort-West Medical Research Centre" of the Ministry of Healthcare of the Russian Federation in 2016 - January 2017. Measurement of glucose concentration and haemolysis in blood plasma was performed in 30 patients.

Comparative results of glucose determination after blood sampling with the proposed device and separation gel containing sodium fluoride and potassium oxalate as an anticoagulant and vials with sodium fluoride and EDTA (according to the closest analogue) is shown in Table 1.
1. EDTA-fluoride-gel
2. Oxalate-fluoride-gel
3. EDTA-fluoride without separation gel (according to the closest analogue)

**Table 1. Haemoglobin level in blood plasma (g / l) after collecting of the donor blood with devices for sample preparation using gel and the closest analogue (EDTA) and potassium oxalate were added as anticoagulants.**

| Blood sample number | Type of the device for preparing blood for determining glucose | | |
|---|---|---|---|
| | EDTA-fluoride-gel | Oxalate-fluoride-gel | According to the closest analogue |
| 1 | 0,8 | 0,6 | 0,3 |
| 2 | 0,5 | 0,2 | 0,1 |
| 3 | 0,4 | 0,2 | 0,2 |
| 4 | 0,8 | 0,2 | 0,7 |
| 5 | 0,9 | 0,3 | 0,6 |
| 6 | 0,4 | 0,3 | 0,3 |
| 7 | 0,5 | 0,2 | 0,1 |
| 8 | 0,1 | 0,1 | 0,0 |
| 9 | 0,8 | 0,6 | 0,3 |
| 10 | 0,8 | 0,5 | 0,5 |
| 11 | 0,6 | 0,2 | 0,2 |
| 12 | 0,9 | 0,4 | 0,6 |
| 13 | 0,5 | 0,3 | 0,6 |
| 14 | 1,4 | 0,4 | 0,4 |
| 15 | 0,3 | 0,2 | 0,2 |

As follows from Table 1, our proposed vials comprising potassium oxalate as an anticoagulant, demonstrated statistically significant lower haemolysis than vials comprising EDTA.

Thus, we consider use of potassium oxalate as an anticoagulant an essential feature of the invention.

In case of free haemoglobin concentration in plasma exceeds 0.8 g/l, haemolysis can already be determined visually, in which case the laboratory personnel shall reject such samples in the normal course of work as unsuitable for analysis.

The present study leads to the conclusion that it is appropriate to centrifuge blood directly at the site of its collection, prior to transport, if it is related to its duration or the possibility of vibration or temperature drop along the way. In this case, the probability of haemolysis is completely excluded.

Preparation of the device for collecting blood from the patient and further investigation of it is carried out as follows.

After blood collection according to the vacuum method, the blood in the device is immediately stirred to dissolve the fluoride and potassium oxalate. The blood is then centrifuged within one hour as of the time of blood sampling at 3000 rpm for 10 minutes to separate the plasma in case the blood is to be transported with the possibility of vibration, shaking, or prolonged storage at room temperature. Determination of glucose in the blood plasma is carried out within the period of up to 24 hours upon storage at room temperature. If the laboratory performing the blood plasma glucose test is located near the blood collection point, the device can be centrifuged directly in the laboratory, after which the device is placed in a biochemical analyzer for analysis. Glucose determination is carried out on any stationary biochemical automatic analyzer by placing the device with a barcode onto board of the analyzer.

The proposed device has a number of advantages in comparison with the other known devices.
- Increases the accuracy of glucose measurement.
- Enables to reject the procedure of transferring the upper plasma layer from the primary vial into another (secondary) vial, thus reducing the risks to the health of the laboratory personnel and the possibility of mistakes due to incorrect labelling of secondary vials.
- The device can be placed in an automatic analyzer for the determination of glucose immediately after centrifugation of the blood without the risk of needle getting clogged due to clots and erythrocytes.
- In the case of centrifugation of blood directly at the site of blood collection, the risk of haemolysis of the sample during storage and transport is excluded.

## Claims

1. The device for preparation of blood sample for determining glucose concentration in blood plasma comprises a vacuum test vial with a barcode, a protective cover of grey colour assembled with a rubber stopper, a mixture of sodium fluoride and anticoagulant applied to the its inner wall, **characterized by** further comprising inert separation gel in the amount of 0.6-1.2 g located at the bottom of the vial as an anticoagulant-potassium oxalate.
